# EUROPÄISCHE PATENTANMELDUNG

(11) **EP 3 824 913 A1**
(43) Veröffentlichungstag der Anmeldung: **26.05.2021**
(21) Anmeldenummer: 19210076.6
(22) Anmeldetag: 19.11.2019
(51) Int. Cl.: A61L 27/44, C08K 3/40

(54) **GLASGEFÜLLTE PAEK-FORMMASSEN**

(71) Anmelder: Evonik Operations GmbH, 45128 Essen (DE)
(72) Erfinder: PERL, Thomas, 60325 Frankfurt am Main (DE); SCHERBLE, Jonas, 64380 Roßdorf (DE); CLAUS, Frank, 55270 Essenheim (DE); KNEBEL, Marc, 68542 Heddesheim (DE); VELTHOEN, Ingrid, 7037 EB Beek (NL)
(74) Vertreter: Evonik Patent Association

(57) **Zusammenfassung**

Die Erfindung ist auf Formmassen enthaltend Polyarylenetherketone und Glaspartikel gerichtet, wobei die Glaspartikel gebrochene irregulär geformte Partikel sind.

## Beschreibung

Die Erfindung ist auf Formmassen enthaltend Polyarylenetherketone und Glaspartikel gerichtet, wobei die Glaspartikel gebrochene irregulär geformte Partikel sind.

Polyarylenetherketone (PAEK) sind von Natur aus etwas grau und ist wenig für ästhetische Anwendungen geeignet. Deshalb wird dem Rohstoff z.B. mittels Compoundierung eine bestimmte Menge Farbstoff hinzugefügt (zum Beispiel Titandioxid, um eine weiße Farbe zu erreichen). Das Material wird entweder in Form von Granulat für Spritzgussanwendungen oder Halbzeugen (extrudierte Vollstäbe) für spanende Bearbeitung angeboten.

Wird das Material im Dentalbereich zum Beispiel für Brückenkonstruktionen genutzt, so ist derzeitig auf dem Markt befindliches PEEK nicht dazu geeignet, für mehrgliedrige Brückenkonstruktionen verwendet zu werden, da es sich durchbiegen würde - es ist also nicht "fest" genug, das heißt, der Zug-Modul des Materials ist nicht hoch genug. Der Zug-Modul der angebotenen Varianten beträgt ca. 3500 MPa (ungefülltes) bis 4100 MPa (farbiges Material).

Gängige Materialien zur Erhöhung des Zugmoduls bei PEEK sind Fasern, zum Beispiel Carbonfasern oder Glasfasern, wie in WO 2006/094690 offenbart. Dies wird im Industriebereich bereits kommerziell eingesetzt. Das Zugmodul lässt sich dadurch zwar signifikant erhöhen. Nachteilig ist allerdings, dass sich das Material nicht mehr homogen verhält und anisotrop wird. Es kann viel Kraft in eine bestimmte Richtung aufnehmen (in Faserrichtung), bereits leichte Winkeländerungen gegenüber der Faserrichtung bewirken allerdings eine drastische Abnahme des Zugmoduls. Für den Medizinbereich ist des Weiteren nachteilig, dass zum einen die Fasern biokompatibel sein müssen und zum anderen die nicht im Material eingekapselten Faserenden Reizungen z.B. der Schleimhäute hervorrufen können.

Um den Anforderungen eines Materials z.B. für mehrgliedrige Brückenkonstruktionen in der Zahntechnik nachzukommen, müsste ein verstärktes Material entwickelt werden. Hierbei ist ein Zug-Modul von mehr als 5500 MPa zu anzustreben. Grundsätzlich lässt sich PEEK durch die Zugabe von Füllstoffen verstärken. Zugesetzte Farbstoffpartikel bewirken eine Verstärkung der Polymermatrix. Es ist bekannt, dass die Festigkeit (ausgedrückt als Zugmodul gemäß EN ISO 527) mit der Menge des zugesetzten Füllstoffs ansteigt. Auf der anderen Seite sinkt durch die Zugabe eines Füllstoffes die Duktilität des Materials. Dies bedeutet, dass das Material mit der Zugabe von Füllstoffen spröder wird und daher bei Belastung früher bricht. Durch einfache Zugabe von zum Beispiel Titandioxid könnte das Zugmodul bis zum gewünschten Grad erhöht werden. Da das Material dann aber zu spröde wäre, ist dies keine Lösungsmöglichkeit.

Aufgabe ist es einen partikulären Füllstoff oder eine Mischung von Füllstoffen zu finden, der einerseits das Zug-Modul erhöht, auf der anderen Seite aber die Duktilität des Materials bestmöglich erhält.

Die Aufgabe wurde gelöst, in dem bestimmte Glaspartikel angewendet wurden.

Gegenstand der Erfindung ist eine Formmasse, die mindestens 30 Gew.-% Polyarylenetherketon (PAEK), bevorzugt 30 bis 80 Gew.-%, mehr bevorzugt 40 bis 70 Gew.-%, insbesondre bevorzugt 50 bis 60 Gew.-% und mindestens zwei Füllstoffe aufweist, wobei einer der Füllstoffe aus Glaspartikeln besteht, wobei die Glaspartikel eine Partikelgrößenverteilung gemäß ISO 13320:2009 d₅₀ von 0,1 µm bis 10 µm aufweisen.

Ein weiterer Gegenstand sind Formkörper enthaltend die erfindungsgemäßen Formmassen.

Ein weiterer Gegenstand ist die Verwendung der erfindungsgemäßen Formkörper als Stützelement.

Die erfindungsgemäßen Formmassen und Formkörper, enthaltend die erfindungsgemäßen Formmassen, sowie die erfindungsgemäße Verwendung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahmen von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden nachfolgend %-Angaben gemacht, so handelt es sich, wenn nicht anders angegeben, um Angaben in Gewichts-%. Bei Zusammensetzungen beziehen sich die %-Angaben, wenn nicht anders angegeben auf die Gesamtzusammensetzung. Werden nachfolgend Mittelwerte angegeben, so handelt es sich, wenn nicht anders angegeben, um Massenmittel (Gewichtsmittel). Werden nachfolgend Messwerte angegeben, so wurden diese Messwerte, wenn nicht anders angegeben, bei einem Druck von 101325 Pa und einer Temperatur von 25 °C ermittelt.

Im Schutzumfang liegen im kommerziellen Handeln übliche Konfektionierungen und Abpackungen der erfindungsgemäßen Produkte. Umfasst ist das Produkt als solches, sowie eventuelle Zerkleinerungsformen (z.B. Malgüter, Extrusionsrohformen wie Granulate, Drähte, Stangen und so weiter) soweit diese nicht in den Ansprüchen definiert sind.

Ein Vorteil der erfindungsgemäßen Formmassen ist, dass die Glaspartikel homogen in der Polymermatrix verteilt sind. Ein weiterer Vorteil ist, dass diese homogene Verteilung ohne Zusatz eines Dispergierhilfsmittels erfolgt.

Ein Vorteil der erfindungsgemäßen Formkörper ist, dass sie bei gleichem Massenfüllgrad (Gew.-%) eine höhere Bruchdehnung und CHARPY Kerbschlagzähigkeit aufweisen, als Formkörper, die aus Formmassen mit Füllstoffen des Standes der Technik wie Titandioxid oder fumed Silica ohne Glaspartikel, hergestellt wurden.

Polyarylenetherketon (PAEK) sind bevorzugt ausgewählt aus Polyetheretherketon (PEEK), Polyetherketon (PEK), Polyetherdiphenyletherketon (PEDEK), Polyetherketonetherketonketon (PEKEKK), Polyetherketonketon (PEKK), Polysulfon (PSU), Polyethersulfon (PES), Polyarylsulfon (PAS), sowie Mischungen und Copolymere daraus. Mehr bevorzugte aromatische Polyether sind Polyetheretherketon (PEEK), Polyetherketon (PEK), Polyetherdiphenyletherketon (PEDEK), Polyetherketonetherketonketon (PEKEKK), Polyetherketonketon (PEKK), weiter mehr bevorzugt sind Polyetheretherketon (PEEK) und Polyetherdiphenyletherketon (PEDEK).

Im Falle von Copolymeren sind die verschiedenen Einheiten des Polyarylenetherketons (PAEK) statistisch verteilt. Statistische Verteilungen sind blockweise aufgebaut mit einer beliebigen Anzahl an Blöcken und einer beliebigen Sequenz oder sie unterliegen einer randomisierten Verteilung, sie können auch alternierend aufgebaut sein oder auch über die Polymerkette einen Gradienten bilden, insbesondere können sie auch alle Mischformen bilden, bei denen gegebenenfalls Gruppen unterschiedlicher Verteilungen aufeinander folgen können. Spezielle Ausführungen können dazu führen, dass die statistischen Verteilungen durch die Ausführung Beschränkungen erfahren. Für alle Bereiche, die nicht von der Beschränkung betroffen sind, ändert sich die statistische Verteilung nicht.

Weiter bevorzugt weisen die Polyarylenetherketone (PAEK) Einheiten der Formel (I) auf

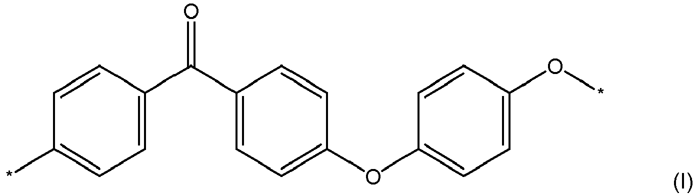

Wobei die Sterne weitere Bestandteile der Polymerkette symbolisieren.Insbesondere bevorzugt ist das Polyarylenetherketone (PAEK) ein Polyetheretherketon (PEEK).

Die Glaspartikel sind massiv, gebrochen und irregulär geformte Partikel.

Das Glas ist ein anorganisches Glas, bevorzugt enthaltend als Vernetzungseinheiten Silicat, Borat und Aluminat. Silicat wird in Produktspezifikationen als SiO₂ angegeben und berechnet, Borat als B₂O₃ und Aluminat als Al₂O₃. Mehr bevorzugt bilden die Silicate die Mehrheit der Vernetzungseinheiten. Derartige Gläser sind dem Fachmann als Alumo-Boro-Silkatgläser bekannt. Weiter bevorzugt ist der Gehalt SiO₂ gleich oder größer als 35 Gew.-%, bevorzugt größer als 45 Gew.-% und besonders bevorzugt gleich oder größer als 50 Gew.-% bezogen auf die Gesamtmasse des Glases, bevorzugt ist der der Gehalt SiO₂ gleich oder kleiner als 95 Gew.-%, 85 Gew.-%, 80 Gew.-% und insbesondere bevorzugt kleiner oder gleich 75 Gew.-%. Bevorzugt ist der Gehalt an Vernetzungseinheiten basierend auf Phosphat (angegeben und berechnet als P₂O₅) kleiner als 5 Gew.-%, bevorzugt kleiner als 2 Gew.-%, bezogen auf den Gehalt im Glas, und insbesondere ist kein Phosphat im Glas enthalten.

Besonders bevorzugt sind Gläser, die
35 bis 85 Gew.-%, 40 bis 75 Gew.-%, bevorzugt 45 bis 55 Gew.-% SiO₂,
5 bis 20 Gew.-%, bevorzugt 9 bis 16 Gew.-% B₂O₃,
0,5 bis 20 Gew.-%, 1 bis 19 Gew.-%, 5 bis 18 Gew.-%, bevorzugt 9 bis 16 Gew.-% Al₂O₃,
0 bis 10 Gew.-% K₂O und
bis zu 40 Gew.-%, bevorzugt 5 bis 30 Gew.-%, insbesondere 10 bis 20 Gew.-% mindestens eines Metalloxides enthalten, wobei die Prozentwerte auf die Gesamtmasse des Glases bezogen sind, Verunreinigungen wie zum Beispiel Verbindungen von Blei, Cadmium, Quecksilber und hexavalentes Chrom betragen maximal 100ppm und bleiben unberücksichtigt. Diese Metalloxide können dem Glas eine gewisse Röngtenopazität verleihen. Geeignete Metalloxide können ausgewählt werden aus BaO, SrO, Cs₂O, SnO₂.

Das Glas beinhaltet SiO₂ mit einem Anteil von 35 bis 85 Gew.-% als glasbildende Komponente. Als SiO₂ -Obergrenze kann bei vorteilhaften Ausführungsformen 73 Gew.-%, bevorzugt 70 Gew.-%, besonders bevorzugt 68,5 Gew.-% gewählt werden. Erfindungsgemäß beträgt die Untergrenze 35 Gew.-%. Niedrigere Gehalte können sich negativ auf die chemische Beständigkeit auswirken.

Verunreinigungen übersteigen in der Regel nicht einen Anteil von 0,2 Gew.-%, insbesondere 0,1 Gew.-%. Dies beinhaltet natürlich auch die vollkommene Freiheit von der jeweiligen Komponente. "frei von einer Komponente" bedeutet also, dass das Glas diese Komponente im Wesentlichen nicht enthält, d.h. dass eine solche Komponente höchstens als Verunreinigung in dem Glas vorliegt, jedoch der Glaszusammensetzung nicht als einzelne Komponente zugegeben wird.

Eine Kontamination des Glases mit den unerwünschten Stoffen soll in der Regel für Fe₂O₃ 300 ppm, bevorzugt höchstens 100 ppm, für PbO 30 ppm, für As₂O₃ 20 ppm, für Sb₂O₃ 20 ppm und für andere Verunreinigungen 100 ppm nicht überschreiten.

Es ist vorgesehen, dass das erfindungsgemäße Glas optional bis auf höchstens Verunreinigungen frei ist von CeO₂ und TiO₂. CeO₂ und TiO₂ verschieben aufgrund Ihrer Absorption im UV-Bereich die UV-Kante des Glases, so dass es eine unerwünschte gelbliche Färbung erhalten kann. In einer bevorzugten Ausführung ist das erfindungsgemäße Glas TiO₂ frei. Eine besonders bevorzugte Ausführung des Glases ist frei von TiO₂ und ZrO₂.

Es ist selbstverständlich auch möglich, die Farberscheinung des Glases für optische oder andere technische Anwendungen durch die Zugabe von dazu gebräuchlichen Oxiden anzupassen. Zur Färbung von Gläsern geeignete Oxide sind dem Fachmann bekannt, beispielsweise seien CuO und CoO genannt, die für diese Zwecke bevorzugt von größer 0 bis 0,5 Gew.-% zugesetzt werden können.

Außerdem kann dem Glas durch Zusätze von z.B. Ag₂O von größer 0 bis 3 Gew.-% eine antiseptische-Funktion verliehen werden. Alkalioxide aus der Gruppe Li₂O, Na₂O, K₂O können benötigt werden, um das Glas überhaupt aufschmelzen zu können. K₂O dient der Einstellung der Einschmelztemperaturen und stärkt gleichzeitig das Glasnetzwerk. Daher ist es erfindungsgemäß mit einem Anteil von 0 bis 10 Gew.-% in der Glaszusammensetzung vorhanden. Bevorzugt ist der Bereich von 0 bis 7 Gew.-%, besonders bevorzugt von 0 bis 5 Gew.-%. Die erfindungsgemäße Obergrenze von 10 Gew.-% sollte nicht überschritten werden, weil der Gehalt an Alkalioxiden die chemische Beständigkeit verringert. Vorteilhaft kann als Obergrenze auch 7 Gew.-%, bevorzugt 5 Gew.-%, besonders bevorzugt 4 Gew.-% gewählt werden.

Natrium- und Lithium-Ionen können aufgrund ihrer geringen Größe leichter aus der Glasmatrix herausgelöst werden, wodurch sich die chemische Beständigkeit, insbesondere die hydrolytische Beständigkeit verringern kann. Bevorzugt sind die Oxide K₂O, Na₂O und Li₂O in Summe zu maximal 6 Gew.-%, bevorzugt max. 5 Gew.-%, mehr bevorzugt max. 4 Gew.-% enthalten. In einer vorteilhaften Ausführung der Erfindung ist das Glas bis auf höchstens Verunreinigungen frei von Li₂O. In einer weiter mehr bevorzugten Ausführung ist das Glas frei von Na₂O und Li₂O.

Bevorzugt sind die Glaspartikel massiv. "Massiv" im Sinne der Erfindung bedeutet, dass die Partikel außer dem Glas maximal 10 Vol.-%, bevorzugt maximal 5 Vol.-%, mehr bevorzugt maximal 2 Vol.-%, besonders bevorzugt maximal 1 Vol.-% und insbesondere bevorzugt keine gasförmigen Einschlüsse aufweist. Der Begriff "massiv" schließt Hohlkörper wie z.B. Glashohlkugeln oder Bubbles bevorzugt aus.

Bevorzugt sind die Glaspartikel gebrochen. "Gebrochen" im Sinne der Erfindung bedeutet, dass die Partikel Produkte aus brechender Bearbeitung sind, wie bevorzugt Mahlgüter. Bevorzugt sind die Glaspartikel irregulär geformt. Die Partikel sind bevorzugt keine sogenannten sphärischen Partikel, wie zum Beispiel Kugeln oder Ellipsoide. Die Partikel sind bevorzugt auch keine Fasermaterialien.

Mehr bevorzugt sind die Glaspartikel massiv und gebrochen, irregulär geformt.

Bevorzugt weisen die Gläser einen Brechungsindex von 1,48 bis 1,56 auf. Der Brechungsindex wird in bekannter Art bestimmt, bevorzugt an einem nicht gebrochenen Körper.

Insbesondere bevorzugt sind sogenannte Dentalgläser.

Bevorzugt weisen die erfindungsgemäßen Formmassen maximal 5 Gew.-%, max. 3 Gew.-%, max 2 Gew.-%, max 1 Gew.-% und insbesondere bevorzugt keine Glaspartikel jedweder Form auf, die außerhalb der hier genannten Spezifikationen liegen.

Alle Gew.-% Angaben der Gläser sind jeweils auf den Gehalt im Glas bezogen.

In einer bevorzugten Ausführungsform wird die Oberfläche des Glaspulvers, d.h. die Oberfläche der Glaspulverpartikel, mit gebräuchlichen Methoden silanisiert. Durch die Silanisierung kann erreicht werden, dass die Bindung der anorganischen Füllstoffe an die Kunststoffmatrix verbessert wird.

Die Glaspartikel weisen eine Partikelgrößenverteilung gemäß ISO 13320:2009 d₅₀ von 0,1 µm bis 10 µm auf, bevorzugt von 0,4 bis 2 µm, insbesondere von 0,5 bis 1,2 µm.

Bevorzugt sind die Glaspartikel frei von Partikeln mit einer Partikelgröße von größer 50 µm, mehr bevorzugt von größer 40µm, weiter mehr bevorzugt 30 µm, besonders bevorzugt 20 µm, weiter besonders bevorzugt 15 µm und insbesondere bevorzugt frei von Partikeln mit einer Partikelgröße größer 10µm.Bevorzugt sind die d₉₉ Werte der Glaspartikel kleiner oder gleich dem vierfachen des d₅₀ Wertes.

Bevorzugt weist die erfindungsgemäße Formmasse 10 bis 50 Gew.-% Glaspartikel auf, bezogen auf die Gesamtformmasse.

Bevorzugt weist die erfindungsgemäße Formmasse weitere Füllstoffe auf. Bevorzugt weist die erfindungsgemäße Formmasse 10 bis 60 Gew.-% an Füllstoffen auf, mehr bevorzugt 20 bis 55 Gew.-%, 30 bis 50 Gew.-% und 40 bis 45 Gew.-%, bezogen auf die Gesamtformmasse.

Bevorzugte Füllstoffe sind TiO₂ und BaSO₄.

Bevorzugt weist die erfindungsgemäße Formmasse mindestens 50 Gew-% Glaspartikel, bevorzugt mindestens 55 Gew.-%, 60 Gew.-%, 65 Gew.-%, 70 Gew.-%und mehr bevorzugt mindestens 75 Gew.-% bezogen auf die Gesamtmasse der Füllstoffe auf.

Die Matrix der erfindungsgemäßen Formmasse, also der Anteil der Formmasse ohne Füllstoffe, enthält 80 bis 100 Gew.-%, bevorzugt 85 bis 99 Gew.-%, mehr bevorzugt 90 bis 95 Gew.-% und insbesondere bevorzugt 90 bis 92 Gew.-% Polyaryletherketon (PAEK), bezogen auf die Gesamtmasse der Polymermatrix.

Neben dem Polyarylenetherketon (PAEK) können weitere Komponenten in der Matrix enthalten sein:
0 bis 5 Gew.-% Röntgenkontrastmittel, bevorzugt 0,1 bis 4 Gew.-%, ausgenommen BaSO₄, welches ein Füllstoff ist,
0 bis 10 Gew.-% Farbstoffe, bevorzugt 0,1 bis 5 Gew-%, mehr bevorzugt 0,2 bis 3 Gew.-%, insbesondere 0,3 bis 1 Gew.-%, ausgenommen TiO₂, welches ein Füllstoff ist,
0 bis 5 Gew.-% impact modifier, bevorzugt 0,1 bis 2 Gew.-%
0 bis 10 Gew.-% andere Polymere,
0 bis 10 % weitere Additive
bezogen auf die Polymermatrix.

Röntgenkontrastmittel können alle Stoffe sein, die zur Anwendung an Mensch und Tier zugelassen sind und die einen entsprechenden Schatten im Röntgenbild verursachen. Bevorzugte Stoffe sind Bariumoxid, Strotiumsulfat und oder Strontiumoxid.

Farbstoffe können alle Stoffe sein, die zur Anwendung an Mensch und Tier zugelassen sind und die eine entsprechende Farbgebung garantieren.

Bevorzugte Farbmittel sind anorganische Pigmente, bevorzugt Metalloxide. Besonders bevorzugt sind, Eisenoxide als Rotpigment und Rutilpigmente für weitere Farben, z.B. Chromtitangelb und Nickeltitangelb als Gelbpigmente. Metalloxide, die bereits als Füllstoff beansprucht wurden sind nicht als anorganisches Pigment zu berücksichtigen.

Weiterhin weisen die erfindungsgemäßen Formmassen bevorzugt weniger als 2 Gew.-%, besonders bevorzugt keine Fasermaterialien, bezogen auf die Gesamtformmasse, auf. Fasermaterialien sind dadurch charakterisiert, dass sie ein Aspektverhältnis von größer 5, mehr bevorzugt von größer 3 aufweisen. Das Aspektverhältnis ist dem Fachmann als Quotient der größten Ausdehnung und der kleinsten Ausdehnung bekannt. Fasermaterialien schließen Glasfasern oder Kohlenstofffasern einschließlich sogenannter Carbon-Nanotubes ein.

Bevorzugt enthalten die erfindungsgemäßen Formmassen PEEK, 10 bis 60 Gew.-% an Füllstoffen, bezogen auf die Gesamtformmasse und mindestens 50 Gew.-% Glaspartikel, bevorzugt mindestens 55 Gew.-%, 60 Gew.-%, 65 Gew.-%, 70 Gew.-%und mehr bevorzugt mindestens 75 Gew.-% bezogen auf die Gesamtfüllstoffmenge.

Mehr bevorzugt enthalten die erfindungsgemäßen Formmassen PEEK, als Füllstoff TiO₂ und mindestens 50 Gew.-% Glaspartikel, bevorzugt mindestens 55 Gew.-%, 60 Gew.-%, 65 Gew.-%, 70 Gew.-%und mehr bevorzugt mindestens 75 Gew.-% bezogen auf die Gesamtfüllstoffmenge.

Weiter mehr bevorzugt enthalten die erfindungsgemäßen Formmassen PEEK, als Füllstoff TiO₂ und mindestens 60 Gew.-% Glaspartikel, wobei das Glas der Glaspartikel die Vernetzungseinheiten Silicat, Borat und Aluminat aufweist (angegeben und berechnet als SiO₂, B₂O₃, Al₂O₃) und bevorzugt einen Gehalt an Vernetzungseinheiten basierend auf Phosphat (angegeben und berechnet als P₂O₅) von kleiner als 5 Gew.-%, bevorzugt kleiner als 2 Gew.-%, bezogen auf den Gehalt im Glas, und insbesondere ist kein Phosphat im Glas enthalten.

Ebenfalls weiter mehr bevorzugt enthalten die erfindungsgemäßen Formmassen PEEK, als Füllstoff TiO₂ und mindestens 50 Gew.-% Glaspartikel, wobei das Glas der Glaspartikel die Vernetzungseinheiten Silicat, Borat und Aluminat aufweist (angegeben und berechnet als SiO₂, B₂O₃, Al₂O₃) und bevorzugt einen Gehalt an Vernetzungseinheiten basierend auf Phosphat (angegeben und berechnet als P₂O₅) von kleiner als 2 Gew.-%, bezogen auf den Gehalt im Glas, und Partikelgrößenverteilungen des Glaspartikel gemäß ISO 13320:2009 d₅₀ von 0,4 bis 2 µm, insbesondere von 0,5 bis 1,2 µm.

Besonders bevorzugt enthalten die erfindungsgemäßen Formmassen PEEK, als Füllstoff TiO₂ und mindestens 50 Gew.-% Glaspartikel, wobei das Glas der Glaspartikel die Vernetzungseinheiten Silicat, Borat und Aluminat aufweist (angegeben und berechnet als SiO₂, B₂O₃, Al₂O₃) und bevorzugt einen Gehalt an Vernetzungseinheiten basierend auf Phosphat (angegeben und berechnet als P₂O₅) von kleiner als 2 Gew.-%, bezogen auf den Gehalt im Glas, und insbesondere ist kein Phosphat im Glas enthalten, wobei die Formmasse bevorzugt weniger als 2 Gew.-%, besonders bevorzugt keine Fasermaterialien, bezogen auf die Gesamtformmasse aufweist.

Ein weiterer Gegenstand der Erfindung ist die Verwendung der erfindungsgemäßen Formmasse zur Herstellung von Medizinprodukten, bevorzugt implantierbaren Knochenprothesen oder als Dentalprothesen.

Die erfindungsgemäße Formmasse wird aus den einzelnen Bestandteilen vorzugsweise durch Schmelzemischen in einem knetenden Aggregat also unter Anwendung von Scherkräften hergestellt.

Die erfindungsgemäßen Formmassen können weitere Additive enthalten.

Bevorzugte Additive sind Oxidationsstabilisatoren, UV-Stabilisator, Hydrolysestabilisatoren, Schlagzähmodifikatoren, Pigmente, Farbstoffe und/oder Verarbeitungshilfsmittel.

In einer bevorzugten Ausführungsform enthalten die Formmassen eine wirksame Menge eines Oxidationsstabilisators und besonders bevorzugt eine wirksame Menge eines Oxidationsstabilisators in Kombination mit der wirksamen Menge eines kupferhaltigen Stabilisators. Geeignete Oxidationsstabilisatoren sind beispielweise aromatische Amine, sterisch gehinderte Phenole, Phosphite, Phosphonite, Thiosynergisten, Hydroxylamine, Benzofuranonderivate, acryloylmodifizierte Phenole etc. Derartige Oxidationsstabilisatoren sind in einer Vielzahl von Typen kommerziell erhältlich, beispielsweise unter den Handelsnamen Naugard 445, Irganox 1010, Irganox 1098, Irgafos 168, P-EPQ oder Lowinox DSTDP. Im Allgemeinen enthalten die Formmassen etwa 0,01 bis etwa 2 Gew.-% und bevorzugt etwa 0,1 bis etwa 1,5 Gew.-% eines Oxidationsstabilisators.

Darüber hinaus können die Formmassen auch einen UV-Stabilisator bzw. einen Lichtstabilisator vom HALS-Typ enthalten. Geeignete UV-Stabilisatoren sind in erster Linie organische UV-Absorber, beispielsweise Benzophenonderivate, Benzotriazolderivate, Oxalanilide oder Phenyltriazine. Lichtstabilisatoren vom HALS-Typ sind Tetramethylpiperidinderivate; es handelt sich hier um Inhibitoren, die als Radikalfänger wirken. UV-Stabilisatoren und Lichtstabilisatoren können vorteilhaft in Kombination eingesetzt werden. Beide sind in einer Vielzahl von Typen kommerziell erhältlich; hinsichtlich der Dosierung kann den Herstellerangaben gefolgt werden.

Die Formmassen können zusätzlich einen Hydrolysestabilisator enthalten wie etwa ein monomeres, oligomeres oder polymeres Carbodiimid oder ein Bisoxazolin.

Weiterhin können die Formmassen Schlagzähmodifikatoren enthalten. Schlagzähmachende Kautschuke für Polyamidformmassen sind Stand der Technik. Sie enthalten funktionelle Gruppen, die von ungesättigten funktionellen Verbindungen herrühren, die entweder in die Hauptkette einpolymerisiert oder auf die Hauptkette aufgepfropft wurden. Am gebräuchlichsten sind EPM- oder EPDM-Kautschuk, der mit Maleinsäureanhydrid radikalisch gepfropft wurde. Derartige Kautschuke können auch zusammen mit einem unfunktionalisierten Polyolefin wie z. B. isotaktischem Polypropylen eingesetzt werden, wie in der EP0683210A2 (US5874176A) beschrieben.

Geeignete Verarbeitungshilfsmittel sind beispielsweise Paraffine, Fettalkohole, Fettsäureamide, Stearate wie Calciumstearat, Paraffinwachse, Montanate oder Polysiloxane.

Erfindungsgemäße Formkörper sind bevorzugt mittels Extrusion hergestellte Halbzeuge. Diese Halbzeuge sind bevorzugt massive Rohlinge, z.B. sogenannte Fräsronden, aus denen nachfolgend spanabhebend die Formstücke, z.B. Prothesen, gefertigt werden.

Weitere erfindungsgemäße Formkörper weisen mindestens eine Schicht hergestellt aus den erfindungsgemäßen Formmassen auf, wobei die Schicht auch ein Teilformkörper sein kann.

Bevorzugt sind die Formkörper vollständig aus den erfindungsgemäßen Formmassen hergestellt.

Bevorzugt weisen die erfindungsgemäßen Formkörper nach DIN EN 527-2, 2012 ein Zugmodul von größer als 5500 MPa auf.

Weiterhin bevorzugt weisen die erfindungsgemäßen Formkörper nach DIN EN 527-2, 2012 ein Zugmodul von größer als 4300 MPa auf und eine Bruchdehnung von mehr als 15 % auf.

Weiter mehr bevorzugt weisen die erfindungsgemäßen Formkörper nach DIN EN 527-2, 2012 ein Zugmodul von größer als 4300 MPa auf und einen Wert X von größer als 100, bevorzugt größer 150 auf. Ein oberer Grenzwert des Wertes X kann 500 sein.

Der Wert X ist definiert als der Betrag des Produktes der Bruchdehnung (gemessen in %) nach DIN EN 527-2, 2012 und der Charpy Kerbschlagzähigkeit (gemessen in kJ/m²) nach DIN 180.

Besonders bevorzugt weisen die erfindungsgemäßen Formkörper nach DIN EN 527-2, 2012 ein Zugmodul von größer als 5500 MPa auf und einen Wert X von größer als 30, bevorzugt größer 40 auf.

Der erfindungsgemäße Formkörper ist vorzugsweise eine Prothese im Dentalbereich. Prothesen sind Teil- oder Vollgebisse, Kronen und Brücken. Hierbei können die Zähne und die verbindende Brücke aus unterschiedlichen Materialien, bevorzugt jedoch aus ein oder mehreren erfindungsgemäßen Formmassen herstellt sein, die sich zum Beispiel in ihrer Färbung unterscheiden.

Die mechanischen Prüfungen sind dem Fachmann bekannt, bevorzugt werden sie nach DIN EN 527-2, 2012 durchgeführt, Bevorzugt werden zur Prüfung sogenannte Schulterstäbe des Typs 1BA eingesetzt. Kerbschlagzähigkeitsprüfungen bevorzugt nach ISO 180 oder nach ISO 179 durchgeführt.

In den Beispielen wurden folgende Komponenten bzw. Formmassen verwendet. VESTAKEEP ist ein Warenzeichen der Evonik, Deutschland und bezeichnet Formmassen auf Basis von Polyetheretherketon mit und ohne Zuschlagstoffe:
Es wurden VESTAKEEP® Dental DC4420 G eine weiß durchgefärbte PEEK Formmasse zur Anwendung im Dentalbereich, VESTAKEEP® Dental DC4450 G eine gelb durchgefärbte PEEK Formmasse zur Anwendung im Dentalbereich, und VESTAKEEP® Dental D4 G verwendet.

Geprüft wurden Gläser unterschiedlicher Zusammensetzung und unterschiedlicher Partikelgrößen.

| | | |
|---|---|---|
| Glas Typ 1 | SiO₂ | ca. 50 Gew.-% |
| | SrO | ca. 20 Gew.-% |
| | B₂O₃ | ca. 15 Gew.-% |
| | Al₂O₃ | ca. 15 Gew.-% |
| | BaO | ca. 1 Gew.-% |
| Glas Typ 2 | SiO₂ | ca. 55 Gew.-% |
| | BaO | ca. 25 Gew.-% |
| | B₂O₃ | ca. 10 Gew.-% |
| | Al₂O₃ | ca. 10 Gew.-% |

Glas 1 wurde mit den Partikelgrößen a) = 0,4 µm, b) = 0,8 µm und c) = 1,0 µm verwendet; Glas 2 mit a) = 0,4 µm, b) = 0,7 µ und c) = 1,0 µm und d) 3,0 µm.

Die Partikelgrößen sind d50 Werte. Die Partikelgrößen der Gläser wurden mittels Laserbeugung mit einem CILAS 1064L gemäß ISO 13320:2009 bestimmt.

| | |
|---|---|
| TiO₂ | Titandioxid |
| Gelbpigment | Chromtitangelb |
| BaSO₄ | Bariumsulfat; |

Formmassen A mit unterschiedlichen Füllgraden.

**Tabelle 1: Zusammensetzung der Formmassen A des Beispiels 1.**

| Formmasse A | TiO₂ [Gew.-%] | BaSO₄ [Gew.-%] | gelb | Gesamt Füllgrad [Gew.-%] | Anteil Glas [Gew.-%] |
|---|---|---|---|---|---|
| 1 | 25,2 | 5,81 | 1,26 | 32,27 | 0 |
| 2 | 29,73 | 5,58 | 1,49 | 36,8 | 0 |
| 3 | 38,1 | 5,14 | 1,9 | 45,14 | 0 |
| 4 | 41,81 | 4,95 | 2,09 | 48,85 | 0 |
| 5 | 45,25 | 4,77 | 2,26 | 52,28 | 0 |
| 6 | 48,46 | 4,6 | 2,42 | 55,48 | 0 |
| 7 | 51,45 | 4,45 | 2,57 | 58,47 | 0 |
| 8 | 10 | 0 | 0 | 20 | 50 |
| 9 | 5 | 0 | 0 | 20 | 75 |
| 10 | 0 | 0 | 0 | 20 | 100 |
| 11 | 10 | 0 | 0 | 25 | 60 |
| 12 | 5 | 0 | 0 | 25 | 80 |
| 13 | 5 | 0 | 0 | 30 | 83,33 |

**Tabelle 2: Zusammensetzung der Formmassen B des Beispiels 1. Die Mengen an betrugen TiO₂ 10,48 Gew.-% und an Chromtitangelb 0,52 Gew.-%**

| Formmasse B | Glas (Typ; Größe) [Gew-%] | Gesamt Füllgrad [Gew.-%] | Anteil Glas [Gew.-%] |
|---|---|---|---|
| 14 | 30 (1; 1µm) | 41 | 73,17 |
| 15 | 30 (1; 0,7µm) | 41 | 73,17 |
| 16 | 30 (1; 0,4µm) | 41 | 73,17 |
| 17 | 28,5 (2; 3µm) | 39,5 | 72,15 |
| 18 | 28,5 (2; 1µm) | 39,5 | 72,15 |
| 19 | 28,5 (2; 0,7µm) | 39,5 | 72,15 |
| 20 | 28,5 (2; 0,4µm) | 39,5 | 72,15 |

### Beispiel 2, mechanische Prüfungen:

Die in den Tabellen 2 dargestellten Messwerte (gemäß DIN EN ISO 527-2, 2012, DIN für Charpy) sind arithmetische Mittelwerte von 5 Formstücken (Zugprüfung) und 10 Formstücken (Schlagprüfung).

Die Bezeichnung der Prüfkörper wurde aus der Bezeichnung der Formmassen übernommen.

**Tabelle 3a: Bestimmung mechanischen Eigenschaften gemäß Beispiel 2; nb bedeutet, dass der Wert nicht bestimmt wurde**

| Prüfkörper A | Zugmodul [MPa] | Charpy | Bruchdehnung [%] | Wert X |
|---|---|---|---|---|
| 1 | 4899 | 6,7 | 11,3 | 76 |
| 2 | 5261 | 6,8 | 9,5 | 65 |
| 3 | 5932 | 5 | 6,5 | 33 |
| 4 | 6468 | 4,7 | 4,2 | 20 |
| 5 | 6963 | 4,4 | 3,4 | 15 |
| 6 | 7453 | 4,2 | 2,4 | 10 |
| 7 | 8258 | 3,5 | 2 | 7 |
| 8 | 4414 | 8,8 | 26 | 229 |
| 9 | 4552 | 9,2 | 17,7 | 163 |
| 10 | 4635 | 8,8 | 24,8 | 218 |
| 11 | 4737 | 9,7 | 21,7 | 210 |
| 12 | 4855 | 10,8 | 20,5 | 221 |
| 13 | 5237 | 11,6 | 18,2 | 211 |

Die Ergebnisse zeigen, dass Dentalglas eine positive Wirkung auf die mechanischen Eigenschaften der Formkörper hat. Es versteift das Material in gleichem Maße (Zugmodul) wie TiO₂-Füllmaterialien, bewahrt dabei aber vergleichsweise höhere Bruchdehnungen.

**Tabelle 3b: Ergebnisse der Testreihe 3**

| Prüfkörper B | Zugmodul [MPa] | Charpy | Bruchdehnung [%] | Wert X |
|---|---|---|---|---|
| 14 | 6085 | 8,1 | 9,1 | 74 |
| 15 | 5952 | 10,3 | 8,9 | 92 |
| 16 | 5989 | 8,3 | 7,4 | 61 |
| 17 | 5953 | 3,4 | 5,6 | 19 |
| 18 | 6078 | 4,5 | 9,2 | 41 |
| 19 | 5996 | 7 | 7,5 | 53 |
| 20 | 5731 | 6,5 | 8 | 52 |

## Patentansprüche

1. Formmasse, die mindestens 30 Gew.-% Polyarylenetherketon (PAEK) und mindestens zwei Füllstoffe aufweist, wobei einer der Füllstoffe aus Glaspartikeln besteht, wobei die Glaspartikel eine Partikelgrößenverteilung gemäß ISO 13320:2009 d₅₀ von 0,1 µm bis 10 µm aufweisen.

2. Formmasse nach Anspruch 1, wobei das Glas der Glaspartikel ein anorganisches Glas ist, enthaltend die Vernetzungseinheiten Silicat, Borat und Aluminat, angegeben und berechnet als SiO₂, B₂O₃, Al₂O₃, und bevorzugt einen Gehalt an Vernetzungseinheiten basierend auf Phosphat, angegeben und berechnet als P₂O₅, von kleiner als 5 Gew.-%, bevorzugt kleiner als 2 Gew.-%, bezogen auf den Gehalt im Glas, und insbesondere ist kein Phosphat im Glas enthalten.

3. Formmasse nach Anspruch 1 oder 2, wobei das Glas in Summe maximal 6 Gew.-%, bevorzugt max. 5 Gew.-%, mehr bevorzugt max. 4 Gew.-% K₂O, Na₂O und Li₂O enthalten, bezogen auf den Gehalt im Glas.

4. Formmasse nach mindestens einem der vorgehenden Ansprüche, wobei die Glaspartikel massiv und gebrochen, irregulär geformt sind.

5. Formmasse nach mindestens einem der vorgehenden Ansprüche, wobei die Glaspartikel weisen eine Partikelgrößenverteilung gemäß ISO 13320:2009 d₅₀ von 0,4 bis 2 µm, insbesondere von 0,5 bis 1,2 µm

6. Formmasse nach mindestens einem der vorgehenden Ansprüche, wobei die Formmasse 10 bis 60 Gew.-% an Füllstoffen aufweist, mehr bevorzugt 20 bis 55 Gew.-%, 30 bis 50 Gew.-% und 40 bis 45 Gew.-%, bezogen auf die Gesamtformmasse.

7. Formmasse nach mindestens einem der vorgehenden Ansprüche, wobei die Formmasse mindestens 50 Gew.-% Glaspartikel, bevorzugt mindestens 55 Gew.-%, 60 Gew.-%, 65 Gew.-%, 70 Gew.-% und mehr bevorzugt mindestens 75 Gew.-% bezogen auf die Gesamtmasse der Füllstoffe aufweist.

8. Formmasse nach mindestens einem der vorgehenden Ansprüche, wobei die Formmasse bevorzugt weniger als 2 Gew.-%, besonders bevorzugt keine Fasermaterialien, bezogen auf die Gesamtformmasse aufweist.

9. Ein weiterer Gegenstand sind Formkörper enthaltend die erfindungsgemäßen Formmassen.

10. Ein weiterer Gegenstand ist die Verwendung der erfindungsgemäßen Formkörper als Medizinprodukt.
